# EUROPEAN PATENT APPLICATION

(11) **EP 2 669 676 A2**
(43) Date of publication of application: **04.12.2013**
(21) Application number: 13169191.7
(22) Date of filing: 24.05.2013
(51) Int. Cl.: G01N 33/18, G01N 27/30, G01N 27/48

(54) **Electrochemical sensor apparatus and electrochemical sensing method**

(30) Priority: 28.05.2012 GB 201209359
(71) Applicant: Process Instruments (UK) Limited, Burnley, Lancashire BB11 4JW (GB)
(72) Inventor: Fielden, Peter Robert, Rossendale, Lancashire BB4 9LB (GB); Goddard, Nicholas John, Manchester, Lancashire M15 5LL (GB); Moorcroft, Stefanie, Salford, Lancashire M28 1FT (GB); Stacey, Craig, Burnley, Lancashire BB10 4HU (GB); Cook, Jonathan Frank, Oldham, Lancashire OL2 8JQ (GB); Riding, Michael Laurence, Burnley, Lancashire BB11 4JW (GB)
(74) Representative: Robinson, Ian Michael

(57) **Abstract**

An electrochemical sensor apparatus and electrochemical sensing method are described, using one or more working electrodes 11, 12 of boron doped diamond (BDD). A cathodic reduction process provides a cathodic measurement and, substantially simultaneously, an anodic oxidation process provides an anodic measurement. A sum of a content of two equilibrium species within an aqueous system is obtained using both the cathodic measurement and the anodic measurement. One example measures total free chlorine by simultaneously measuring hypochlorous acid (HOCl) and hypochlorite ion (OCI-).

## Description

### BACKGROUND

### Technical Field

The present invention relates in general to the field of electrochemical sensor apparatus and electrochemical sensing methods. In particular, but not exclusively, the invention relates to an apparatus and method to measure an aqueous solution containing a disinfectant such as chlorine.

### Description of Related Art

It is well known to use chlorine as a water additive. For example, chlorine is applied for disinfection of swimming pools, for treating drinking water, or during food processing. Hence, there is a general need for a chlorine analyser to measure the presence of chlorine in an aqueous solution. Such chlorine analysers are widely needed for measurement in environmental or industrial situations.

Known measurement techniques to monitor chlorine in water on-line are usually based on a wet chemical reagent and optical measurement, or an electrochemical probe. US2005/029103 (Feng et al) describes an example chlorine sensor of the related art which measures a chlorine species by electrochemical analysis.

The known chlorine analysers are strongly sensitive to the pH level of the solution being measured. Therefore, typically, a separate measure of the pH level must be taken in order to calibrate the measurements of the chlorine analyser. It would be desirable to avoid this need for a second sensor to measure pH. Also, the typical chlorine analyser is constructed to include a buffer (e.g. a solution or gel) that stabilises pH of the water sample within a measurement chamber. However, it has been noted that the buffer introduces several disadvantages, such as complication of the instrument and delay in achieving a measurement, and thus it would be desirable to avoid the need for a buffer.

Generally, it is desired to address one or more of the disadvantages associated with the related art, whether those disadvantages are specifically discussed herein or will be otherwise appreciated by the skilled person from reading the following description. In particular, it is desired to provide an electrochemical sensor apparatus and an electrochemical sensing method which is simple, reliable and cost-effective.

### SUMMARY OF THE INVENTION

According to the present invention there is provided an electrochemical sensor apparatus and electrochemical sensing method as set forth in the appended claims. Other features of the invention will be apparent from the dependent claims, and the description which follows.

According to an aspect of the present invention there is provided an electrochemical sensing method suitable for measuring an aqueous system. The method includes measuring a cathodic reduction process using a working electrode of boron doped diamond (BDD) to provide a cathodic measurement, measuring an anodic oxidation process using a BDD working electrode to provide an anodic measurement, and outputting a result indicating a sum of a content of two equilibrium species within the aqueous system using both the cathodic measurement and the anodic measurement.

According to an aspect of the present invention there is provided an electrochemical sensor apparatus. The apparatus includes at least one working electrode of boron doped diamond (BDD). A measurement unit is arranged to measure a cathodic reduction process to provide a cathodic measurement using a working electrode of boron doped diamond (BDD), and to measure an anodic oxidation process to provide an anodic measurement also using a BDD working electrode. A processing unit is arranged to output a result indicating a sum of a content of two equilibrium species within the aqueous system using both the cathodic measurement and the anodic measurement.

As will be discussed in more detail below, the example embodiments address many of the difficulties of the related art. At least some examples provide a simple, reliable and effective mechanism for measuring chlorine species in aqueous solutions.

In one example, the anodic and cathodic measurements may be performed consecutively at a single BDD working electrode. In another example, the anodic and cathodic measurements may be performed at two or more separate working electrodes, respectively. Surprisingly, it has been found that problems associated with the pH susceptibility of measurements may be overcome by performing these two related anodic and cathodic measurements substantially simultaneously. That is, the anodic and cathodic measurements are suitably performed at the same time, or consecutively within a relative short space of time, in relation to substantially the same measurement sample.

In one example there is provided an electrochemical sensor apparatus and electrochemical sensing method for measuring a disinfectant in an aqueous solution.

In one example there is provided an electrochemical sensor apparatus and electrochemical sensing method for measuring chlorine as a disinfectant.

In one example, the method and apparatus may be arranged to measure at least one chlorine atom present in aqueous solutions for their disinfectant properties. Suitable examples of molecules comprising at least one chlorine atom include hypochlorous acid, the hypochlorite ion, chlorine dioxide and the chlorite ion.

In one example, there is significant interest in measuring the total free chlorine in chlorinated water, as the combination of hypochlorous acid (HOCl) and the hypochlorite ion (OCl-). Suitably, HOCl is measured by the cathodic measurement, while substantially simultaneously also measuring OCl- by the anodic measurement. Being two equilibrium species, the total free chlorine is the sum of HOCl and OCl-. The relative proportions of these species varies significantly by the measurement pH, while the [HOCl] / [OCl-] ratio is constant for any particular pH. Thus, in the example embodiments, summing the measured concentrations of HOCl and OCl- provides the total free chlorine. Notably, the mechanism is independent of measurement pH.

In another example, chlorine dioxide and chlorite are measured by the anodic and cathodic measurements. In this case, chlorine dioxide is measured by the cathodic (reduction) process, and chlorite is measured by the anodic (oxidation) process.

In one example, buffering to control the measurement pH is not required. Instead, the measurements may be performed at any suitable pH. The measurements may be performed over a wide range within the ultimate pH limits of either the reduction and/or oxidation processes occurring in the anodic and cathodic measurements.

In one example, the method may be performed without the presence of a reagent. Typically, a reagent such as perchlorate would be required. Although the perchlorate ion seems to enhance the peak shape of the anodic response to the OCl- species, surprisingly it has now been found that it is unnecessary to include perchlorate in order to obtain a quantitative response.

In one example, the working electrodes are bare working electrodes. The working electrodes may be presented directly to the aqueous system being measured. For example, a wall jet configuration of the sensor apparatus is now possible. A measurement chamber or porous membrane now are not required, leading to a significantly simpler apparatus in some embodiments.

These and other features and advantages will be appreciated further from the following example embodiments.

### BRIEF DESCRIPTION OF THE DRAWINGS

For a better understanding of the invention, and to show how example embodiments may be carried into effect, reference will now be made to the accompanying drawings in which:
Figure 1 is a perspective view of an example chlorine sensor apparatus;
Figure 2 is a sectional plan view of the chlorine sensor;
Figure 3 is a flowchart as a schematic overview of an example method of measuring chlorine;
Figure 4 is a graph of speciation of chlorine in water as a function of pH;
Figure 5 is a graph of a cyclic voltammetric scan of a gold working electrode as a comparative example;
Figure 6 is a graph showing the cathodic and anodic response of a BDD working electrode towards free chlorine;
Figure 7 is a graph showing the cathodic response at a BDD working electrode in more detail;
Figure 8 is a graph showing the anodic response at a BDD working electrode in more detail;
Figure 9 is a graph showing the cathodic response of a platinum working electrode towards dissolved oxygen;
Figure 10 is a graph showing the cathodic response of a BDD working electrode towards dissolved oxygen;
Figure 11 shows the cathodic response of a gold working electrode towards dissolved oxygen as a comparative example;
Figure 12 is a graph illustrating measurement of chlorite and chlorine dioxide; and
Figures 13A-13C are a series of graphs showing calibration data for anodic response of the BDD working electrode to dissolved chlorine at different selected potentials.

### DETAILED DESCRIPTION OF THE EXAMPLE EMBODIMENTS

The example embodiments will be described with reference to a chlorine sensor apparatus and method, particularly to measure total free chlorine. The example embodiments described below relate to the measurement of HOCl and OCl-. In another example, chlorite and chlorine dioxide may be measured. The apparatus and method may be applied in many specific implementations, as will be apparent to persons skilled in the art from the teachings herein.

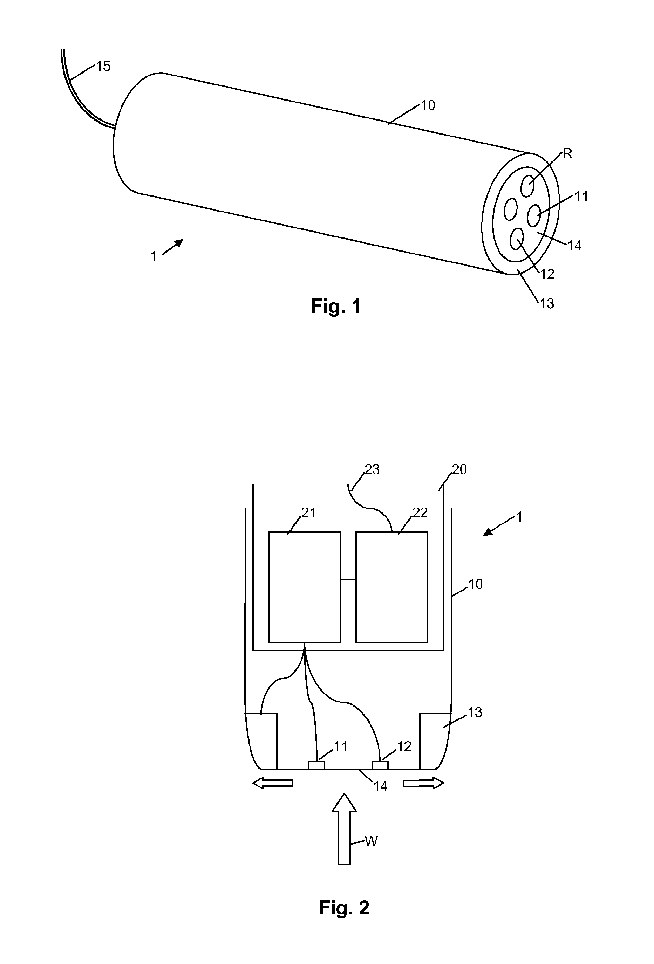

Figure 1 is a perspective view of an example chlorine sensor apparatus 1. In this example, the sensor apparatus 1 comprises a main body or housing 10 having one or more working electrodes 11, 12 at a working surface thereof. A counter electrode 13 may be provided. A reference electrode R may also be provided. Optionally further electrodes may be provided.

In this example, the housing 10 is generally cylindrical and the working surface 14 is provided at one end face of the cylinder. The chlorine sensor is arranged to perform electrochemical analysis. Conveniently, the sensor obtains and processes measurements using the working electrodes 11, 12 and outputs a result or data signal by an appropriate communication path. In this example, the sensor housing 10 is provided with a wired output connection 15 which allows the sensor to be connected or coupled as part of a measurement and control system. Other physical configurations are also envisaged as will be familiar to those skilled in the art. For example, in a wall-jet configuration, it would be appropriate to place a single working electrode at or about the geometric centre of the generally circular working surface. It is also envisaged to use concentric working ring electrodes, with a central disc electrode as a "ring-disc" configuration within a wall-jet flow geometry.

Figure 2 is a sectional view of the example sensor 1 through the sensor body 10. In this example, the counter electrode or auxiliary electrode 13 is provided as an annular ring at the working surface 14 surrounding the working electrodes 11, 12. This example apparatus has bare working electrodes 11, 12 which are directly exposed to a flow of water to be sampled. In n this example the sensor is provided in a 'wall jet' configuration. A flow of water W approaches substantially perpendicular to the measuring surface 14 and is disbursed across the measuring surface to encounter, inter alia, the working electrodes 11, 12 and the auxiliary electrode 13. Notably, in this example, it is not necessary to provide the working electrodes 11, 12 within a separate chamber or provide a porous membrane which separates the electrodes from the main flow of the sample W.

As shown in Figure 2, the sensor housing 10 suitably includes a signal processing unit 20 which is electrically coupled to the electrodes 11, 12, 13, etc. A measuring unit 21 contains circuitry which performs electrochemical analysis using these electrodes. An output unit 22 prepares a data signal 23 to be output from the sensor apparatus, such as via the wire 15. It will be appreciated that many other specific configurations of the apparatus are also possible. For example, the signal processing unit 20, the measuring unit 21 and/or the output unit 22 may be provided remote from the main sensor housing 10, the number, the physical configuration of the electrodes 11, 12, 13 may be changed, and so on.

In one example embodiment, only one working electrode 11 is required, leading to a simpler and smaller configuration of the device. In another example, two separate working electrodes 11, 12 are provided, which may allow improved measurements. Suitably, these working electrodes comprise boron doped diamond (BDD). Doped diamond has been developed as a versatile electrode material and has been studied in some detail over the past years. However, several additional interesting and surprising advantages for BDD electrodes have now been identified, particularly in the context of chlorine measurement.

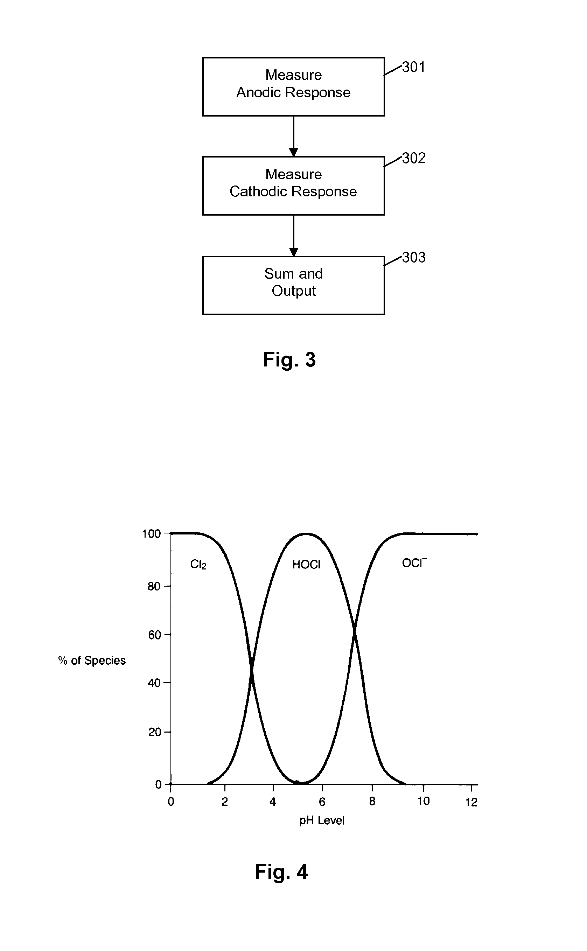

Figure 3 is a flowchart as a schematic overview of an example method of measuring chlorine.

Step 301 comprises measuring an anodic oxidation process to provide an anodic measurement. This step is performed using any first one of the one or more working electrodes 11, 12.

Step 302 comprises measuring a cathodic reduction process to provide a cathodic measurement. Step 302 may be performed again by the first electrode 11 consecutively before or after the step 301. Alternately, the step 302 may be performed by a separate second working electrode 12. Conveniently, the steps 301 and 302 are performed in close temporal proximity, e.g. at the same time or within a few seconds of each other, so as to capture measurements in relation to substantially the same sample.

Step 303 comprises outputting a result indicating a sum of a content of two equilibrium species within the aqueous system using both the cathodic measurement and the anodic measurement.

It will be appreciated that the anodic and cathodic measurements of steps 301 and 302 occur when a relevant potential difference is applied to induce a current flow through the working electrode. In a typical configuration of the sensor, the counter electrode 13 is biased relative to the relevant working electrode 11, or vice versa, while the other is held at or near ground potential. In voltammetry, and particularly in an amperometric system, the current is measured as a function of time and is indicative of the concentration of the species being measured.

As will be familiar to those skilled in the art, chlorine dissolves in water and establishes the equilibria described by equations 1 and 2 below:

Cl₂ + H₂O ↔ HCl + HOCl (Hypochlorous acid) (eqn. 1)

HOCl ↔ H⁺ + OCl⁻ (Hypochlorite ion) (eqn. 2)

Two key species that are present in chlorinated water are hypochlorous acid and the hypochlorite ion. The relative proportions of chlorine and these species is controlled principally by the pH of the water. This is illustrated in Figure 4, which shows how these proportions are distributed over the range pH 0 to pH 12. See also "Residual Chlorine - A guide to measurement in water applications", Stephen Russell, WRc Instrument Handbooks, WRc plc, Swindon, Figure 2, Page 4, 1994. (ISBN 1 898920 17 6).

The usual range of pH associated with potable water is such that the principal species present in solution are hypochlorous acid and hypochlorite ion. It should be noted that at about pH 5, the speciation is uniquely hypochlorous acid alone, and that above circa pH 9, the hypochlorite ion predominates.

The crossing point of the HOCl and OCl- curves occurs at pH 7.54 at 25 °C. This pH dependency of chlorine speciation is influential, both in terms of the optimisation of disinfection, and when consideration is given to the measurement of dissolved chlorine as a process monitoring variable. Hypochlorous acid has been recognised to be the most effective disinfection agent of the dissolved chlorine species.

The chemical speciation in chlorine disinfected water becomes more complicated when there is a coincident source of ammonia and related nitrogen compounds. This leads to the formation of chloramines, through the following sequential reactions:

HOCl + NH₃ → NH₂Cl + H₂O (monochloroamine) (eqn. 3)

NH₂Cl + HOCl → NHCl₂ + H₂O (dichloamine) (eqn. 4)

NHCl₂ + HOCl → NCl₃ + H₂O (trichloramine) (eqn. 5)

These three reactions are a significant simplification of the likely reality in chlorinated potable water. The presence of organic nitrogen sources, such as proteins (which break down to yield amino acids), further complicate the chemistry of the chloramines. Hence, measuring chlorine in water is not straightforward. In the related art, Free Chlorine is typically used to describe the sum of the concentrations of the inorganic chlorine species in the water (HOCl and OCl⁻). Combined Chlorine includes the sum of the concentrations of the nitrogenous chlorine species in the water (chloramines), and Total Chlorine is usually taken as the sum of the free chlorine and combined chlorine species.

Within a sensing system based on reductive amperometry there is the possibility of interference due to the presence of dissolved oxygen within the sensing solution, or in a supporting electrolyte/buffer if used. Dissolved oxygen is known to follow a two-step reduction process at the cathode, which will be observed as two distinct voltammetric reduction waves. A first step of the general type O + n₁e → R₁ is a two electron reduction, where the H₂O₂ generated is the reduction product, R₁:

O₂ + 2H₂O + 2e → H₂O₂ + 2OH⁻ (eqn. 6)

A second step of the general type R₁ + n₂e → R₂ usually occurs at significantly more cathodic (negative) potentials:

H₂O₂ + 2e → 2OH⁻ (eqn. 7)

Hence, there is a desire to reduce this interference by dissolved oxygen.

Conventional free chlorine measurement probes evaluate the HOCl concentration by electrochemical reduction (at a cathode working electrode) via the following reaction:

HOCl + 2e⁻ → Cl⁻ + OH⁻ (eqn. 8)

The OCl⁻ species cannot undergo reduction, so does not register at the cathode working electrode. The current which is measured at the cathode working electrode is due to the flux of the electrons supplied from the electrode to promote the reaction in equation 8. The electron flux, and hence the measured current, is a function mainly of HOCl concentration and electrode area. Since the electrode area is fixed, the current should be proportional to HOCl concentration at the surface of the cathode working electrode. The concentration of HOCl is also a function of solution pH, according to the following equation where species concentration is represented by [HOCl] and [OCl⁻ ] respectively:

log [HOCl] / [OCl⁻ ] = pKₐ - pH (eqn. 9)

The acid dissociation constant, *pKₐ*, as a function of temperature, *T* (K) is found by the approximation:

*pKₐ* = 3000/*T* - 10.0686 + 0.0253T (eqn. 10)

This adds complexity to the typical measurement process, since a change in the measurement solution pH will result in a change in the ratio of HOCl species concentration to OCl⁻ species concentration. As the pH increases, the concentration of free HOCl in solution decreases, and the concentration of free OCl⁻ in solution increases. The usual way to remove the experimental variable of pH dependency is to control the pH at the cathode working electrode by immersing it in a pH buffer (a chemical reagent that fixes the pH at a predetermined level). From the speciation plot in Figure 4, a pH 5 buffer would tend to maximise the free solution concentration of HOCl and minimise the concentration of OCl⁻.

As noted above, the example embodiments employ a dual measurement mechanism using BDD working electrodes to identify the respective species independently of pH, in particular to overcome the pH susceptibility of cathodic amperometric free chlorine measurements. The dual measurements are characterised by the substantially simultaneous measurement of both a cathodic (reduction) and an anodic (oxidation) process. In this example of free chlorine measurement, the cathodic reaction already described and as used in conventional free chlorine measurement probes, will be used in conjunction with the anodic reaction that may be used to monitor the OCl⁻ species. The reaction involved is described by:

6ClO⁻ + 3H₂O → 2ClO₃⁻ + 4Cl⁻ + 6H⁺ + 3/2O₂ + 6e⁻ (eqn. 11)

The simultaneous quantitative measurement of both HOCl and OCl⁻ at the same time allows the determination of free chlorine at any pH, since the free chlorine will be the sum of the concentrations of HOCl and OCl⁻. Thus, the measurement could be buffered to control the measurement pH, but could equally well measure at any pH (within the ultimate pH limits of either the reduction and/or oxidation processes).

This simultaneous measurement of the two species (HOCl and OCl⁻) might be achieved using a range of electrode materials (traditionally, platinum, gold, or carbon and, particularly, glassy carbon). However, a potential limitation with these traditional electrode materials is their potential range. At the extremes of their cathodic range, protons in the solution will lead to a background current, according to the reaction:

2H⁺ + 2e⁻ → H₂ (eqn. 12)

At the extremes of their anodic range, hydroxyl ions in the solution will lead to a background current, according to the two-stage reaction:

2OH⁻ - 2e⁻ → H₂O₂ (eqn. 13)

Then,

H₂O₂ - 2e⁻ → 2H⁺ + O₂ (eqn. 14)

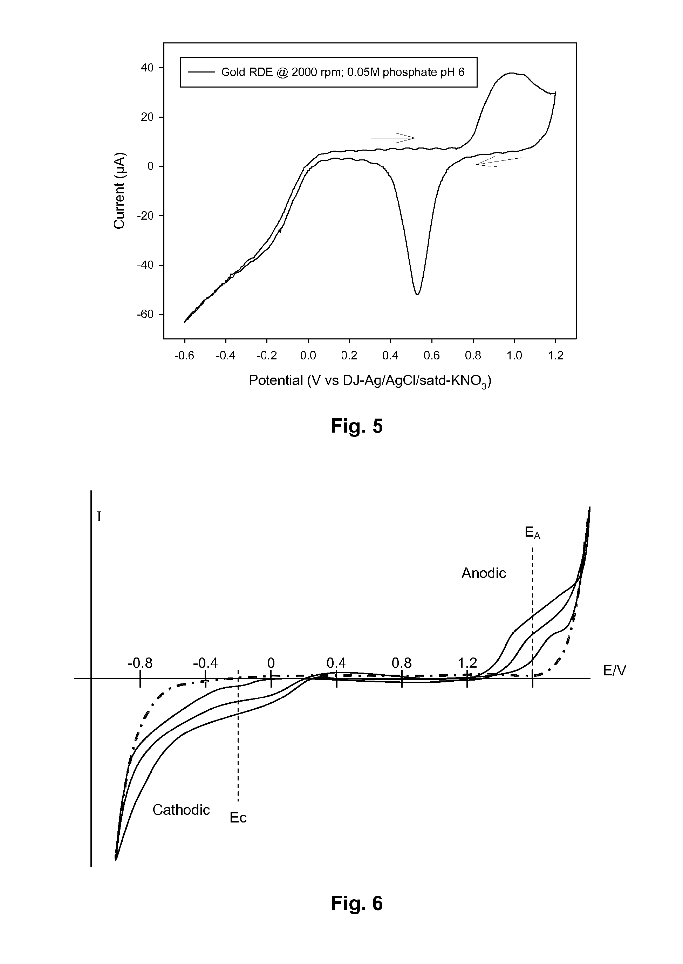

Unfortunately, the reality is more complex, since noble metal electrodes are prone to oxide layer formation at high anodic potentials. This may be illustrated in Figure 5 for a gold working electrode. Figure 5 is a cyclic voltammetric scan of a gold working electrode (rotating disc electrode, at 2000 rpm), in a pH 6 phosphate buffer solution, as a comparative example.

As shown in Figure 5, the anodic current rises significantly at an anodic potential more positive than about +0.8 V (vs reference electrode), as characterised by the current "hump". The negative peak at +0.55 V (vs reference electrode), is the reduction of the oxide surface back to gold as the potential is scanned in the cathodic direction. Clearly, such oxide film formation renders a noble metal electrode unsuitable for operation at any anodic potential more positive than the potential associated with the onset of surface oxidation. The negative (cathodic) current at potentials more negative than +0.1 V (vs reference electrode) in this example is due to the reduction of dissolved oxygen in the solution, according to the reaction given above. Similar characteristics may be observed for platinum electrodes, and glassy carbon electrodes are noted for their lack of reproducibility and gradual passivation when operated at high anodic potentials. It is, therefore, difficult to utilise traditional electrode materials for sustained measurement experiments at high anodic potentials, such as would be required for the oxidation of the species OCl⁻.

Meanwhile, a simultaneous quantitative measurement of both HOCl and OCl⁻ can actually be achieved by using boron doped diamond (BDD) as the working electrode. BDD has an extremely low native background current over a very wide potential window in both cathodic and anodic directions.

It has been considered to monitor the species OCl⁻ through anodic measurement at a BDD working electrode, but previous examples have consistently employed a highly oxidising supporting electrolyte that contains the perchlorate ion. By contrast, although the presence of the perchlorate ion seems to enhance the peak shape of the anodic response to the OCl⁻ species, surprisingly it has now been found to be unnecessary to include perchlorate in order to obtain a quantitative response.

Figure 6 shows the cathodic and anodic response of a BDD working electrode towards free chlorine. Figure 6 also shows typical applied potentials that could be employed to make cathodic (E_{C}) and anodic (E_{A}) amperometric measurements. Figure 6 summarises the approximate response of a BDD electrode to successive additions of free chlorine (thin solid lines), against its background current (dash-dotted line). The plot represents the response at pH 7.54 (i.e. the pH that corresponds to the pKa of HOCl, where HOCl and OCl⁻ species are in 1:1 equilibrium). As pH increases from 7.54, the HOCl (cathodic) response will diminish and the OCl⁻ (anodic) response will increase. The converse is true as the pH is reduced from 7.54. Indeed, the relative responses will conform to the species equilibrium described above. Thus, the arithmetic sum of the cathodic response with the anodic response will indicate the total free chlorine in the solution.

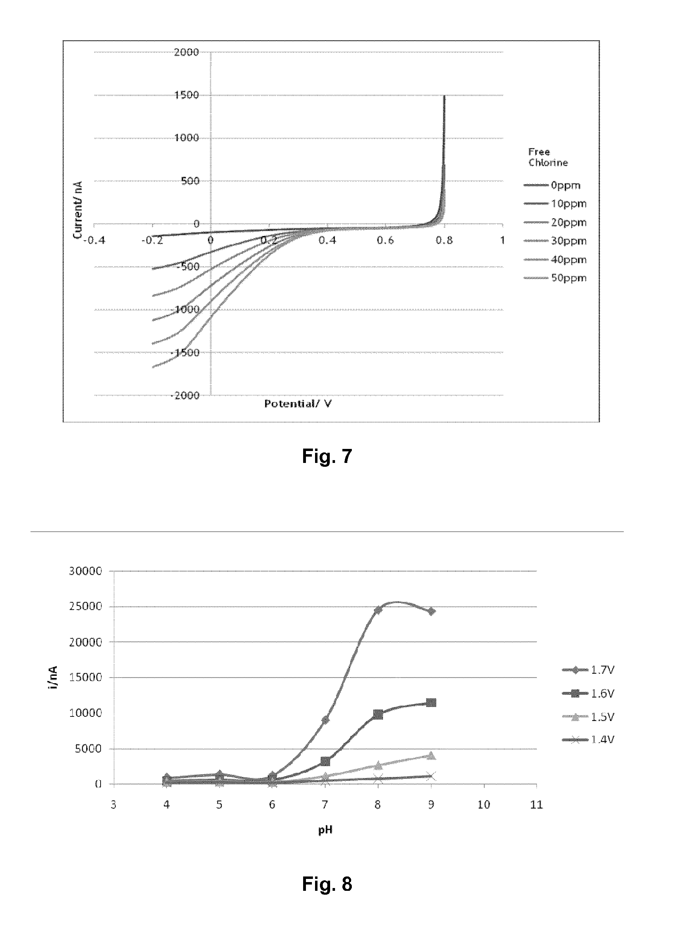

Figure 7 shows the cathodic response at a BDD working electrode to sample solutions loaded at specific concentrations of free chlorine. In this particular experiment, Figure 7 shows the cathodic response of a BDD working electrode towards free chlorine with supporting electrolyte: 0.05M phosphate buffer at pH6; electrode rotated at 1000 rpm; linear sweep at 0.05 Vs⁻¹.

Figure 8 shows the anodic response at a BDD working electrode to sample solutions loaded at a specific concentration of free chlorine, with varied pH and anodic potential at which the current has been measured. In this experiment, Figure 8 shows the anodic response of a BDD working electrode towards free chlorine, over a range of pH and at different anodic potentials. The electrode was rotated at 1000 rpm; linear sweep at 0.05 Vs⁻¹.

Generally, the measuring steps may be performed by a sweep or scan across a voltage range. Measurement samples may be taken periodically during the sweep or scan. The sweep or scan may be linear, or may be cyclical. For some species it may be appropriate to firstly scan to determine the presence of peaks (which may vary for example based on PH or temperature) and then determine the most appropriate measurement points within the scan or sweep.

These experimental examples have demonstrated the link between cathodic measurement of the HOCl species and the anodic measurement of the OCl⁻ species. It also seems that BDD is less prone to interference from the presence of dissolved oxygen in the sample. This is less important for a membrane mediated amperometric probe, since a steady state will be achieved such that any background current due to dissolved oxygen will be constant and small. However, this would not be the case for membraneless systems, where sudden fluctuations in dissolved oxygen will affect the measurement current of the probe system. Notably, a bare electrode chlorine sensor is now feasible.

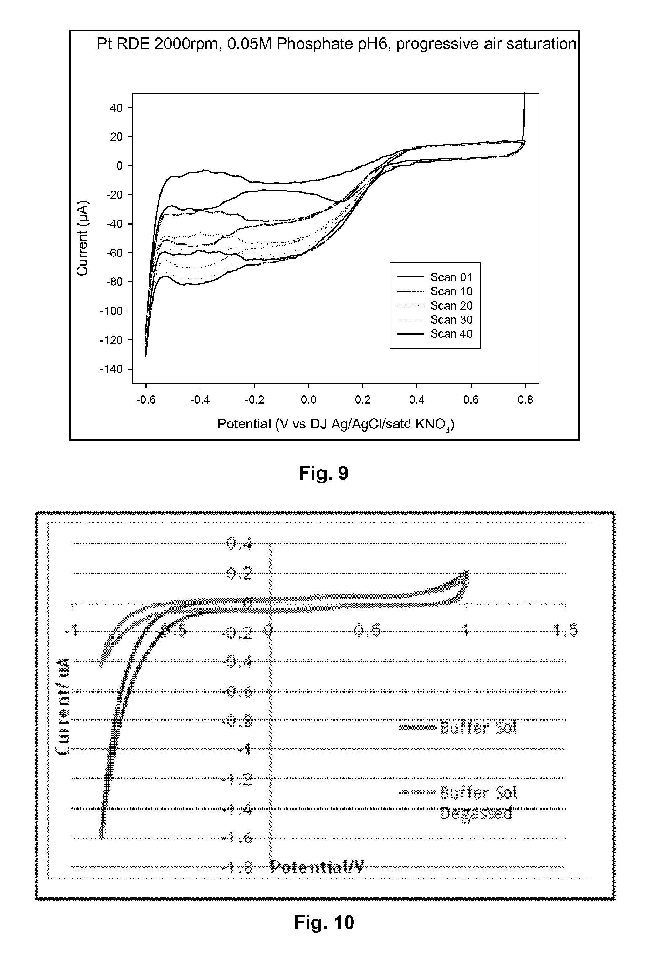

Figure 9 shows the cathodic response of a platinum working electrode towards dissolved oxygen. For comparison, the effect of dissolved oxygen on the background response of a platinum working electrode is shown. The scan numbers are at fixed intervals with exposure of the sample buffer to laboratory air. Scan 01 is the background after dissolved air/oxygen had been expelled from the sample by sparging with helium. Here, the initial measurement (scan 01) is in air/oxygen free buffer, and is therefore the background current for the platinum electrode in the pH 6 phosphate buffer. Subsequent scans are monitored as the solution is progressively exposed to laboratory air. Scan 40 represents the steady state response to the buffer after it has reached equilibration with the laboratory air. Subsequent scans would appear superimposed on the Scan 40 plot.

Figure 10 shows the cathodic response of a BDD working electrode towards dissolved oxygen. Figure 10 is a plot of a degassed and an air saturated buffer solution (0.5M lithium ethanoate, pH5). It is clear from these data that not only is the background less affected by the dissolved oxygen, but also that the background current is substantially less. (Compare the current scales: Platinum 0 to -140 µA; BDD 0 to -1.8 µA).

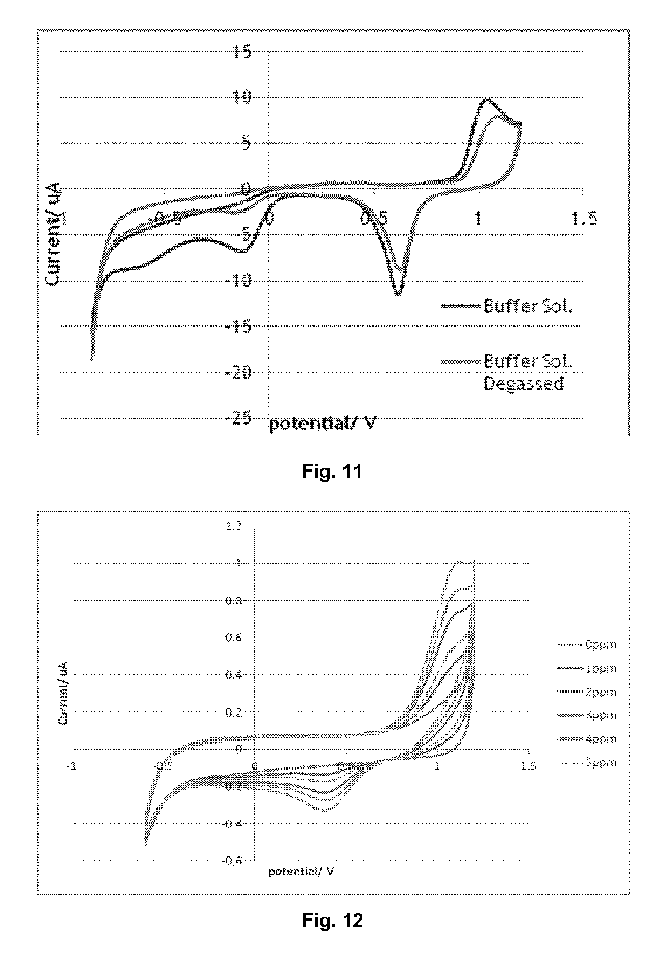

Figure 11 shows the cathodic response of a gold working electrode towards dissolved oxygen as a comparative example. For the sake of completeness, a similar plot is shown in Figure 11 for a gold working electrode with the same electrolyte as used in Figure 10, as a direct comparison between gold and BDD. The difference in current scales should again be noted. (It should be noted that the peaks at +1.0V (anodic) and +0.6V (cathodic) are the oxidation of the gold surface and the reduction of gold oxide respectively).

The principle has been illustrated and exemplified with reference to free chlorine measurement where there are two distinct species that make up an equilibrium composition that is pH dependent. The purpose of making two measurements is to overcome pH sensitivity that is inherent in the speciation chemistry of any sample under observation, where deliberate fixing of the pH through buffering is either undesirable, infeasible, or has only partial effectiveness.

Other possible assays include similar equilibrium coupling of species that occur and are governed by pH. Also, the simultaneous measurement of systems that are self-reversible may be candidates for this approach. An example of this that is of significance to water quality monitoring are the species chlorine dioxide and chlorite, which are related through the following reaction:

ClO₂ + e⁻ → ClO₂⁻ (eqn. 15)

A BDD working electrode may be used to measure chlorine dioxide through its cathodic reduction to the chlorite ion, and also used to measure the chlorite ion through its anodic oxidation to chlorine dioxide. Thus, a single electrode may be used to monitor both species, simply through the control of the applied potential. Similarly to the free chlorine measurement, both chlorine dioxide and chlorite ion may be measured simultaneously by using a combination of a cathodic and anodic assay.

Figure 12 is a graph showing data for chlorite anodic oxidation (topside curves) at ca. +1.0V. This process of chlorite oxidation generates chlorine dioxide, which accumulates at a stationary (no flow, no stirring) BDD working electrode. The reduction of the accumulated chlorine dioxide is clearly visible on the cathodic measurement (underside curves) at ca. +0.4V. Note the response is less for the chlorine dioxide, since the bulk solution contains the chlorite, but it is only the chlorine dioxide that remains near the electrode surface that can be measured in this experiment. The concentrations are the bulk values for chlorite.

At the cathode (reduction - addition of electron), chlorine dioxide is reduced to chlorite as shown in Equation 15 above. At the anode (oxidation - removal of electron), chlorite is oxidised to chlorine dioxide:

ClO₂⁻ → ClO₂ + e⁻ (eqn. 16)

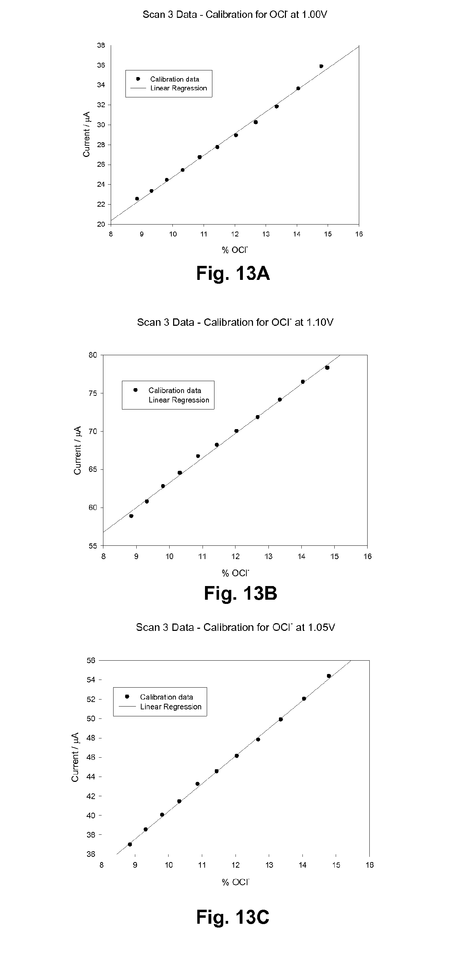

Figures 13A-13C are a series of graphs showing calibration data for anodic response of the BDD working electrode at different selected potentials. In a further enhancement, it has been found that the anodic response of the BDD electrodes exhibits observable nonlinearity compared with an ideal linear regression. The response curve as illustrated in Figure 13A and Figure 13B is typically a sigmoid. Interestingly, the sigmoid deviates around an ideal linear response and the direction of deviation reflects to an opposing direction as the voltage is varied. It has been found that the sensor apparatus may be calibrated by adjusting the applied potential to produce a substantially linear response at or about the point where this deviation inverts. When the potential is lower than ideal, as in Figure 13A, then the sigmoid deviates in one mode, and when too high, as in Figure 13B, deviation is observed in an opposing mode. Between these ranges lies a potential which produces a more or less linear response, as is illustrated in Figure 13C. Thus, the method suitably includes the step of calibrating the anodic potential E_{A} by observing a reverse in the mode of the sigmoid response curve. In the example embodiments, the measuring unit 21 may perform such a corresponding calibration function.

The industrial application of the present invention will be clear from the discussion above. The advantages of the invention have also been discussed and include providing a simple, reliable and efficient mechanism for sensing chlorine species. In some embodiments, a pH buffer or a reagent are not required.

Although a few preferred embodiments have been shown and described, it will be appreciated by those skilled in the art that various changes and modifications might be made without departing from the scope of the invention, as defined in the appended claims.

## Claims

1. An electrochemical sensing method suitable for measuring an aqueous system, the method comprising:
measuring a cathodic reduction process using a working electrode (11, 12) of boron doped diamond to provide a cathodic measurement;
measuring an anodic oxidation process using a boron doped diamond working electrode (11, 12) to provide an anodic measurement; and
outputting a result indicating a sum of a content of two equilibrium species within the aqueous system using both the cathodic measurement and the anodic measurement.

2. The method of claim 1, comprising performing the measuring steps substantially simultaneously with respect to one measurement sample.

3. The method of claim 1 or 2, comprising performing the anodic and cathodic measurements at separate boron doped diamond working electrodes (11, 12), respectively.

4. The method of claim 1, comprising performing the measuring steps consecutively at a single boron doped diamond working electrode (11, 12).

5. The method of any preceding claim, comprising measuring hypochlorous acid (HOCl) by the cathodic measurement and hypochlorite ion (OCl⁻) by the anodic measurement.

6. The method of any preceding claim, comprising outputting a result indicating total free chlorine in chlorinated water, as a combination of measured hypochlorous acid (HOCl) and hypochlorite ion (OCl⁻).

7. The method of any preceding claim, comprising measuring chlorine dioxide by the cathodic measurement and chlorite by the anodic measurement.

8. The method of any preceding claim, comprising performing both measuring steps without buffering to control a measurement pH.

9. The method of any preceding claim, comprising performing both measuring steps without the presence of a reagent.

10. The method of any preceding claim, wherein the working electrodes are bare working electrodes which are presented directly to the aqueous system being measured.

11. The method of any preceding claim, further comprising the step of calibrating a potential applied in the anodic measurement by observing a reversal in a mode of a sigmoid shaped response with respect to varying test potentials.

12. An electrochemical sensor apparatus, comprising:
at least one working electrode (11, 12) of boron doped diamond;
a measurement unit (21) arranged to measure a cathodic reduction process to provide a cathodic measurement using the at least one working electrode (11, 12) of boron doped diamond, and to measure an anodic oxidation process to provide an anodic measurement also using the at least one working electrode (11, 12) of boron doped diamond; and
a processing unit (arranged to output a result indicating a sum of a content of two equilibrium species within an aqueous system using both the cathodic measurement and the anodic measurement.

13. The apparatus of claim 12, wherein the measuring unit performs the cathodic measurement and the anodic measurement consecutively both on the same working electrode (11, 12).

14. The apparatus of claim 12, wherein the measuring unit performs the cathodic measurement and the anodic measurement at the same time on at least two respective working electrodes (11, 12).

15. The apparatus of claim 12, 13 or 14, comprising a housing (10) having in a working surface (14) which presents the one or more working electrodes (11, 12) in a wall-jet configuration.
